# DEMANDE DE BREVET EUROPEEN

(11) **EP 1 179 333 A1**
(43) Date de publication de la demande: **13.02.2002**
(21) Numéro de dépôt: 01440244.0
(22) Date de dépôt: 30.07.2001
(51) Int. Cl.: A61K 6/02

(54) **Composition naturelle destinée à ameliorer la stabilité et la plasticité des masses céramique dentaire avant la mise en forme et leur cuisson**

(30) Priorité: 08.08.2000 FR 0010416
(71) Demandeur: Breuel Ekkehart, 67250 Soultz sous Forêt (FR)
(72) Inventeur: Breuel Ekkehart, 67250 Soultz sous Forêt (FR)

(57) **Abrégé**

Composition naturelle destinée à ameliorer la stabilité et la plasticité de masse de céramique dentaire avant la mise en forme e leur cuisson.

L'objectif de cette découverte est de produire un bio-liquide de modelage avec des substances absolument naturelles, en évitant tout effet secondaire pour l'utilisateur ou son ouvrage, en améliorant tout de même le maniement.

Le résultat des élement naturels comme Eau déminéralisée 96,91%, Silicate de Soude (Na₂SiO₃) et Sorbate de Potassium (CH₃-CH=CH-CH=CH-COOK) 0,09% (ce qui veut dire sans chimie) et d'une meilleure combinaison avec céramique, conduit à une meilleure stabilité et plasticité de la céramique, malgré des temps de cuissons dentine réduit. Cette composition est sans aucun danger pour l'utilisateur ou son ouvrage, ni pour l'environnement

Cette composition, bio-liquide de modelage, est destinée à tous les ceramistes dentaires dans les laboratoires et les cabinets dentaires.

## Description

La présente invention est une composition à utiliser dans les laboratoires et les cabinets dentaires, pour rendre modelable la céramique dentaire.

Dans le liquide de modelage utilisées actuellement, on emploie en règle générale des additifs chimiques (par ex.: chlorure, acide acétique, alcools) afin de donner plus de stabilité et de plasticité aux masses céramiques.Plus la stabilité et la plasticité sont recherchées, plus on inclut de la chimie. Ce qui entraîne une température de preséchage plus longue. Ce temps de pré-séchage à une températur de four de 600°C, peut aller jusqu'à 30 minutes, avec un température de cuisson maximale de 900°C. La composition et la températur de cuisson de la céramique dentaire n'est pas uniforme en raison de la grande variété de ce produit. C'est selon du producteur de la céramique dentaire. Le ingredient principale de la céramique dentaire c'est toutefois 70% feldspath (SiO₂). Pour appliquer la céramique réduire en poudre en fait une mélange avec le liquide de modelage à l'aide d'un pinceau.

La nouvelle composition développée, améliore fondamentalement la stabilité et la plasticité par rapport à celles utilisées actuellement sur le marché. La stabilité et une bonne plasticité sont des critères très importants et déterminants pour chacune des réalisations conforme à la réalité, applicable avec un pinceau humide.

Cette composition permet un temps de pré-séchage très réduit, en fonction de la céramique,ainsi que l'abaissement de la températur de depart de 500°C à 590°C .
De part ses composants absolutement naturels ce bio-liquid est parfaitement écologique. Les substances sont, selon la céramique Eau de deminéralisée 70% à 98%, Silicate de Soude (Na₂SiO₃)de 0,1% à 25% et Sorbate de Potassium (CH₃-CH=CH-CH=CH-COOK) de 0,01% à 10%. En raison de la composition en ce naturelle, et du fait que les substances sont utilisée en tant que médicament par la médecine il ni a aucun risque pour le céramiste dentaire ou l'ouvrage.
De part sa composition naturelle de:
Eau deminéralisée 96,91%, Silicate de Soude (Na₂SiO₃)3,00% et Sorbate de Potassium (CH₃-CH=CH-CH=CH-COOK) 0,09%

En outre il s'est avéré lors des essais, que la température de cuisson maximale de la céramique a pu être réduite, et ainsi diminuer le risque de déformation de l'ouvrage lors de la cuisson dentine, tout particulièrement pour les alliages de métaux précieux.
Cette même composition ce caractérise aussi par une températur de cuisson réduite et une températur de départ de 500°C aprés un temps de pré-séchage de 3 minutes. La température de cuisson final pourrait être abaissée de 10°C par ce procède. Le risque de déformation durant la phase de cuisson est minisé, tout particulièrement pour les alliages dentaire en métaux précieux. Cette composition permet une bonne stabilité et un bonne modelage de la céramique,avec un pinceau.
La mise en oeuvre est plus précise, plus rapide et plus saine. C'est une composition absolument bio-compatible, ainsi adaptée aux allergiques.
Un avantage essentiel s'est avéré lors des essais, les différentes couleurs des couronnes et bridges sont rendues à la fin de la cuisson dentine, plus naturelles et plus intenses avec l'utilisation du nouveau liquide lors de la cuisson dentine. Une combinaison naturelle entre la céramique (ca. 70% feldspath - Sio₂) et Silicate de Soude (Na₂SiO₃) s'établit.

C'est un produit 100% à base naturelle. Ces ingrédientes sont utilisés en médecine général.

On trouve le Silicate de Soude dans la composition de certains médicaments, par exemple dans ceux prévus pour les soins des rhumatismes. Potassium est utilisée pour la conservation des denrées alimentaire. De ce fait aucun danger n'est craindre pour la santé ni pour l'environnement, il est non polluant.

En l'etat des connaissance actuelle, aucun liquide à base naturelle pour modelage céramique est sur le marché à ce jour.Pour les raisons citées précédement cette composition convient également aux personnes souffrant d'allergies.

Cette composition permet de simplifier le travail du céramiste dentaire en évitent tout risque de santé pour l'utilisateur, de préserver l'ouvrage et l'environnement, et tente d'améliorer la performance dans le domaine des dentes artificielles.

## Revendications

1. Composition naturelle destinée à ameliorer la stabilité et la plasticité de masse de céramique dentaire avant la mise en forme et leur cuisson, **caracterisée en ce Qu'**elle est constituée de melange d'Eau démineralisée dans une proportion de 70% à 98%, de Silicate de Soude (Na₂SiO₃) dans une proportion de allont 0,1% à 25% et de Sorbate de Potassium (CH₃-CH=CH-CH=CH-COOK) dans une proportion de 0,01% à 10%.

2. Composition selon la revendication 1 **caracterisée en ce qu'**elle est constituée d'Eau démineralisée dans une proportion de 96,91%, de Silicate de Soude (Na₂SiO₃) dans une proportion de 3,00% et de Sorbate de Potassium (CH₃ -CH=CH-CH=CH-COOK) dans une proportion de 0,09%.
